# FASCICULE DE BREVET EUROPEEN

(11) **EP 2 935 605 B1**
(45) Date de publication et mention de la délivrance du brevet: **11.01.2017**
(21) Numéro de dépôt: 13803153.9
(22) Date de dépôt: 18.11.2013
(51) Int. Cl.: C12P 19/00, C12P 19/12, C12P 21/00, C12N 9/42, C12P 7/10, C12P 7/16

(54) **PROCÉDÉ DE PRODUCTION D'OLIGOSACCHARIDES A PARTIR DE BIOMASSE LIGNOCELLULOSIQUE.**
VERFAHREN ZUR HERSTELLUNG VON OLIGOSACCHARIDEN AUS LIGNOCELLULOSEHALTIGER BIOMASSE
METHOD FOR PRODUCING OLIGOSACCHARIDES FROM A LIGNOCELLULOSE BIOMASS

(30) Priorité: 20.12.2012 FR 1203536
(43) Date de publication de la demande: 28.10.2015
(73) Titulaire: IFP Énergies nouvelles, 92852 Rueil-Malmaison Cedex (FR); Institut National de la Recherche Agronomique (INRA), 75338 Paris Cedex 07 (FR); Agro Industrie Recherches et Developpements, 51110 Pomacle (FR)
(72) Inventeur: JOURDIER, Etienne, F-92420 Vaucresson (FR); AYMARD, Caroline, F-69002 Lyon (FR); BEN CHAABANE, Fadhel, F-75020 Paris (FR)
(86) Numéro de dépôt international: PCT/FR2013/052772
(87) Numéro de publication internationale: WO 2014/096586

(56) Documents cités:
- US-A1- 2004 121 446
- US-A1- 2009 061 486
- US-A1- 2012 064 579
- MARGARITA V. SEMENOVA ET AL: "Disaccharide Synthesis by Enzymatic Condensation of Glucose: Glycoside Linkage Patterns for Different Fungal Species", OPEN GLYCOSCIENCE, vol. 2, no. 1, 19 août 2009 (2009-08-19), pages 20-24, XP055076482, ISSN: 1875-3981, DOI: 10.2174/1875398100902010020
- WARZYWODA M ET AL: "PRODUCTION AND CHARACTERIZATION OF CELLULOLYTIC ENZYMES FROM TRICHODERMA REESEI GROWN ON VARIOUS CATBON SOURCES", BIORESOURCE TECHNOLOGY, ELSEVIER BV, GB, vol. 39, no. 2, 1 janvier 1992 (1992-01-01), pages 125-130, XP008054567, ISSN: 0960-8524, DOI: 10.1016/0960-8524(92)90130-P
- LO C M ET AL: "Cellulase production by continuous culture of Trichoderma reesei Rut C30 using acid hydrolysate prepared to retain more oligosaccharides for induction", BIORESOURCE TECHNOLOGY, ELSEVIER BV, GB, vol. 101, no. 2, 1 January 2010 (2010-01-01), pages 717-723, XP026662968, ISSN: 0960-8524, DOI: 10.1016/J.BIORTECH.2009.08.056 [retrieved on 2009-09-22]

## Description

La présente invention s'inscrit dans le cadre d'un procédé de production de d'oligosaccharides à partir de biomasse lignocellulosique. L'invention a également pour objet un procédé de production de cellulases intégrant une unité de production d'oligosaccharides synthétisés à partir de biomasse lignocellulosique. Enfin la présente invention concerne un procédé dit de "seconde génération" de production de sucres et d'alcools, de solvants ou d'acides organiques à partir de biomasse lignocellulosique et qui comprend une unité de production in-situ d'oligosaccharides et de cellulases.

### Etat de la technique

Afin de répondre aux enjeux de la transition énergétique et notamment pour réduire l'impact des transports sur l'environnement et leur dépendance au pétrole, de nombreuses études sont actuellement menées pour utiliser et optimiser les bioressources renouvelables, comme la biomasse lignocellulosique.

La biomasse lignocellulosique représente une des ressources renouvelables les plus abondantes sur Terre. Les substrats considérés sont très variés, puisqu'ils concernent à la fois les substrats ligneux (feuillus et résineux), les sous-produits de l'agriculture (paille) ou ceux des industries génératrices de déchets lignocellulosiques (industries agroalimentaires, papeteries).

La biomasse lignocellulosique est composée de trois principaux constituants : la cellulose (35 à 50%), l'hémicellulose (23 à 32%) qui est un polysaccharide essentiellement constitué de pentoses et d'hexoses et la lignine (15 à 25%) qui est une macromolécule de structure complexe et de haut poids moléculaire, provenant de la copolymérisation d'alcools phénylpropénoïques. Ces différentes molécules sont responsables des propriétés intrinsèques de la paroi végétale et s'organisent en un enchevêtrement complexe.

La cellulose, majoritaire dans cette biomasse, est ainsi le polymère le plus abondant sur Terre et celui qui présente le plus grand potentiel pour former des matériaux et des biocarburants. Cependant le potentiel de la cellulose et de ses dérivés n'a pas pu, pour le moment, être complètement exploité, majoritairement en raison de la difficulté d'extraction de la cellulose. En effet, cette étape est rendue difficile par la structure même des plantes. Les verrous technologiques identifiés à l'extraction et à la transformation de la cellulose sont notamment son accessibilité, sa cristallinité, son degré de polymérisation, la présence de l'hémicellulose et de la lignine.

Le principe du procédé de conversion de la biomasse lignocellulosique par des procédés biotechnologiques utilise une étape d'hydrolyse enzymatique de la cellulose contenue dans les matières végétales pour produire du glucose. Le glucose obtenu peut ensuite être fermenté en différents produits tels que des alcools (éthanol, 1,3-propanediol, 1-butanol, 1,4-butanediol, ...) ou des acides (acide acétique, acide lactique, acide 3-hydroxypropionique, acide fumarique, acide succinique, ...).

La cellulose et éventuellement les hémicelluloses sont les cibles de l'hydrolyse enzymatique mais ne sont pas directement accessibles aux enzymes. C'est la raison pour laquelle ces substrats doivent subir un prétraitement précédant l'étape d'hydrolyse enzymatique. Le prétraitement vise à modifier les propriétés physiques et physico-chimiques du matériau lignocellulosique, en vue d'améliorer l'accessibilité de la cellulose emprisonnée au sein de la matrice de lignine et d'hémicellulose.

L'hydrolyse est une opération difficile qui met généralement en jeu une hydrolyse du type enzymatique. En effet cette dernière est préconisée car elle génère peu d'effluents à traiter par rapport à une hydrolyse du type chimique (e.g. acide).

A l'heure actuelle, cette hydrolyse est réalisée en présence de cellulases qui sont produites à partir de microorganismes tels que des bactéries ou des champignons (*Clostridium, Aspergillus, Trichoderma*). Ces micro-organismes produisent un cocktail d'enzymes qui agissent en synergie pour hydrolyser la cellulose en monomère glucose, et aussi éventuellement l'hémicellulose. Parmi les familles d'enzymes présentes, on note particulièrement les endoglucanases, les exoglucanases et les β-glucosidases. Parmi ceux-ci, *Trichoderma reesei* est l'espèce la plus prometteuse car elle est capable de sécréter des quantités importantes de cellulases très actives.

Cependant, le coût de production des cellulases reste élevé et représente un des obstacles économiques pour une mise en oeuvre de tels procédés à l'échelle industrielle.

Industriellement, la production optimisée de cellulases par *Trichoderma reesei* est réalisée en protocole fed-batch (alimentation sans soutirage) en utilisant une solution d'alimentation contenant du lactose comme sucre inducteur de la production de cellulases (FR 2555603). Cependant le lactose utilisé seul est trop coûteux pour permettre une production de cellulases à bas prix.

Afin de diminuer le coût associé à l'achat du lactose, il est possible d'envisager de remplacer en totalité ou en partie la fraction de l'inducteur lactose par un autre sucre inducteur, qui serait :
- soit aussi bon inducteur mais moins coûteux
- soit plus coûteux mais meilleur inducteur, ce qui permettrait de diminuer encore la teneur minimale d'inducteur dans la solution d'alimentation du protocole fed-batch.

Ainsi par exemple le cellobiose (dimère de glucose en β(1→4)) est un inducteur naturel de la production de cellulases chez *Trichoderma. reesei.* Le cellobiose est le dernier intermédiaire dans l'hydrolyse de la cellulose en glucose qui est catalysée par l'enzyme β-glucosidase. Afin de produire le cellobiose, on peut songer à utiliser un cocktail de cellulases dépourvu de β-glucosidases afin d'hydrolyser la cellulose en cellobiose. Cependant cette réaction est de cinétique lente car les cellulases (cellobiohydrolase et endoglucanase) sont très fortement inhibées par le cellobiose. Par ailleurs une telle option nécessiterait de produire séparément un cocktail spécifique dépourvu de β-glucosidase, ce qui de fait la rend industriellement moins rentable.

Un autre alternative consisterait à utiliser du sophorose (dimère de glucose en β(1→2)) qui est le plus fort inducteur connu mais son coût reste encore prohibitif pour être utilisé industriellement.

Un but de l'invention est donc de proposer des procédés de production de sucres, de cellulases et d'alcool et/ou de solvants qui soient optimisés du point de vue du coût d'exploitation, notamment à partir de biomasse lignocellulosique.

Le document US2004/0121446 divulgue un procédé de production d'une solution inductrice pour la production de cellulases par un champignon filamenteux. Il est exemplifié la production d'une solution inductrice contenant du sophorose, du gentiobiose et du glucose à partir d'une solution stérilisée de 60% w/w de glucose, par incubation à 65 °C pour 3 jours en présence d'une préparation de cellulase entière.

Le document Lo C.-M. et al., Bioresource Technology, vol. 101, no. 2, 1 janvier 2010, pages 717-723, divulgue la production de cellulases par culture en continu sur un hydrolysat acide comprenant des oligosaccharides. Cet hydrolysat est obtenu par courte hydrolyse acide de sciure de bois (bouillonnement pour 15 minutes avec de l'acide sulfurique).

### Résumé de l'invention

Selon un premier aspect de l'invention, il est proposé un procédé de production d'oligosaccharides à partir de biomasse lignocellulosique comprenant au moins les étapes suivantes :
a) on prétraite dans un réacteur de prétraitement la biomasse de manière à fournir un effluent contenant un substrat prétraité;
b) on effectue dans un réacteur une hydrolyse enzymatique du substrat prétraité contenu dans l'effluent issu de l'étape a) en présence de cellulases de manière à produire un hydrolysat contenant du glucose, des cellulases et de l'eau;
c) on prélève au moins une partie de l'hydrolysat issu de l'étape b) comprenant une fraction liquide;
d) on réduit la teneur en eau de ladite partie de l'hydrolysat prélevé à l'étape c) de sorte que la fraction liquide de l'hydrolysat présente une teneur en eau inférieure à 65% poids d'eau par rapport au poids total de la fraction liquide de l'hydrolysat;
e) on incube à une température comprise entre 40 et 70°C l'hydrolysat issu de l'étape d) pendant le temps nécessaire pour produire un effluent enrichi en oligosaccharides.

Le procédé de production d'oligosaccharides selon l'invention utilise comme matière première de la biomasse lignocellulosique qui est accessible de manière abondante et qui est en outre peu coûteuse.

Les inventeurs ont observé qu'il est possible de promouvoir la formation d'oligosaccharides à partir d'un hydrolysat contenant du glucose et des cellulases par incubation lorsque la teneur en eau de la fraction liquide de l'hydrolysat est inférieure ou égale à 65% poids par rapport au poids total de la fraction liquide de l'hydrolysat.

De préférence l'hydrolysat issue de l'étape d'hydrolyse qui est mise en incubation possède une activité β-glucosidase d'au moins 1 IU/mL, et de préférence d'au moins 5 IU/mL. Cette activité β-glucosidase est mesurée en utilisant comme substrat du *p*NPG (*para-*nitrophénol-β-glucopyranose) à 5mM, dans du tampon citrate 50mM à pH 4,75, avec incubation 30 minutes à 50°C [Dashtban, Maki, Leung, Mao, and Qin (2010) Cellulase activities in biomass conversion: Measurement Methods and Comparison. Critical Reviews in Biotechnology 30 (4) pages 302-309*].* On mesure ensuite par absorbance la concentration en *para*-nitrophénol, produit de l'hydrolyse du *p*NPG par la β-glucosidase. L'activité β-glucosidase est exprimée en IU/mL, où IU signifie µmol/min (pour µmol de produit libéré par minute de réaction).

Selon un mode de réalisation, l'étape d) de réduction de la teneur en eau de la fraction liquide de l'hydrolysat est réalisée par évaporation à une température inférieure à 90°C.

Selon un autre mode de réalisation, l'étape d) de réduction de la teneur en eau de la fraction liquide de l'hydrolysat est effectuée en ajoutant du glucose dans l'hydrolysat prélevé à l'étape c).

De façon alternative, à l'étape d) on sépare l'hydrolysat prélevé à l'étape c) en au moins une première portion et une seconde portion, on concentre la première portion d'hydrolysat à une température supérieure à 90°C de manière à obtenir une portion d'hydrolysat concentré et on mélange l'hydrolysat concentré avec la seconde portion d'hydrolysat non concentré.

Selon un autre mode de réalisation, à l'étape d) on effectue une séparation membranaire de l'hydrolysat prélevé à l'étape c), par exemple par osmose inverse ou nanofiltration, de sorte à récupérer un hydrolysat dont la teneur en eau est réduite et une solution aqueuse.

Il est également possible dans le cadre du procédé de production d'oligosaccharides selon l'invention d'effectuer une séparation solide/liquide de l'effluent issu de l'étape a) de manière à récupérer une fraction liquide contenant des sucres et une fraction solide contenant le substrat prétraité qui envoyée à l'étape b). Quant à la fraction liquide, celle-ci peut être soit traitée en mélange avec l'hydrolysat à l'étape d), soit être mélangée avec l'effluent enrichi en oligosaccharides issu de l'étape e).

De préférence à l'issue de l'étape e) d'incubation, on obtient un effluent comprenant des oligosaccharides choisi parmi le sophorose et le gentobiose seul ou en mélange.

Le procédé de production d'oligosaccharides selon l'invention peut lui-même être intégré dans une unité existante de production d'alcool et/ou de solvants, dites de seconde génération, qui utilise comme matière première de la biomasse lignocellulosique.

Ainsi l'invention a également pour objet un procédé de production de cellulases à partir de biomasse lignocellulosique comprenant au moins les étapes suivantes :
a) on prétraite dans un réacteur de prétraitement la biomasse de manière à fournir un effluent contenant un substrat prétraité;
b) on effectue dans un réacteur une hydrolyse enzymatique du substrat prétraité contenu dans l'effluent issu de l'étape a) en présence de cellulases de manière à produire un hydrolysat contenant du glucose, des cellulases et d'eau;
c) on prélève au moins une partie de l'hydrolysat issu de l'étape b) comprenant une fraction liquide;
d) on réduit la teneur en eau de ladite partie de l'hydrolysat prélevé à l'étape c) de sorte que la fraction liquide de l'hydrolysat présente une teneur en eau inférieure à 65% poids d'eau par rapport au poids total de la fraction liquide de l'hydrolysat;
e) on incube à une température comprise entre 40 et 70°C l'hydrolysat issu de l'étape d) pendant le temps nécessaire pour produire un effluent enrichi en oligosaccharides;
f) on envoie au moins une partie de l'hydrolysat enrichi en oligosaccharides dans un réacteur contenant un milieu de culture et des microorganismes capables de produire les cellulases; et
g) on met en culture le mélange de manière à produire un effluent enrichi en cellulases.

Selon l'invention, l'effluent issu de l'étape e) contenant les oligosaccharides est utilisé comme solution inductrice dans une unité de production de cellulases.

L'invention se rapporte en outre à un procédé de production d'alcools, de solvants ou d'acides organiques seuls ou en mélange, qui comprend les étapes du procédé de production de cellulases tel que décrit plus haut et des étapes supplémentaires dans lesquelles on envoie une partie de l'hydrolysat issu de l'étape b) dans une réacteur de fermentation comprenant des microorganismes afin de produire un moût de fermentation comprenant des alcools, des solvants ou des acides organiques seuls ou en mélange et on recycle au moins une partie de l'effluent enrichi en cellulases issu de l'étape g) dans le réacteur de l'étape b).

Alternativement, le procédé de production d'alcools, de solvants ou d'acides organiques seuls ou en mélange selon l'invention peut mettre en oeuvre une étape dans laquelle on envoie une partie de l'effluent issu de l'étape a) dans une unité d'hydrolyse et de fermentation simultanées afin de produire des alcools et/ou des solvants et on recycle au moins une partie de l'effluent enrichi en cellulases issu de l'étape g) dans l'unité d'hydrolyse et de fermentation simultanées.

La fermentation selon l'invention permet par exemple de produire de l'éthanol comme alcool majoritaire ou du n-butanol seul ou en mélange avec de l'acétone ou de l'iso-propanol.

Le procédé de production d'alcool et/ou de solvants selon l'invention comprend une étape de production in-situ de cellulases à partir d'oligosaccharides qui sont eux-mêmes synthétisés de manière in-situ à partir de sucres déjà présents dans le procédé.

Le procédé de production d'alcool, de solvants ou d'acides organiques seuls ou en mélange selon l'invention présente l'avantage que sa mise en oeuvre s'affranchit des coûts d'achat, de transport et de stockage d'oligosaccharides (utilisés comme inducteurs) nécessaires à la production des cellulases car grâce au procédé les oligosaccharides sont produits in-situ à partir de sucres qui sont déjà disponibles au sein du procédé.

### Brève description de la figure

D'autres caractéristiques et avantages de l'invention seront mieux compris et apparaîtront clairement à la lecture de la description faite ci-après en se référant aux dessins parmi lesquels :
- la figure 1 est une représentation schématique d'un mode de réalisation du procédé de production d'oligosaccharides selon l'invention,
- la figure 2 est une représentation schématique d'un mode de réalisation du procédé de production de cellulases selon l'invention,
- la figure 3 est une représentation schématique d'un premier mode de réalisation du procédé de production d'alcools et/ou de solvants selon l'invention.
- la figure 4 est une représentation schématique d'un second mode de réalisation du procédé de production d'alcools et/ou de solvants selon l'invention.

Généralement, les éléments semblables sont dénotés par des références identiques dans les figures.

### Description détaillée de l'invention

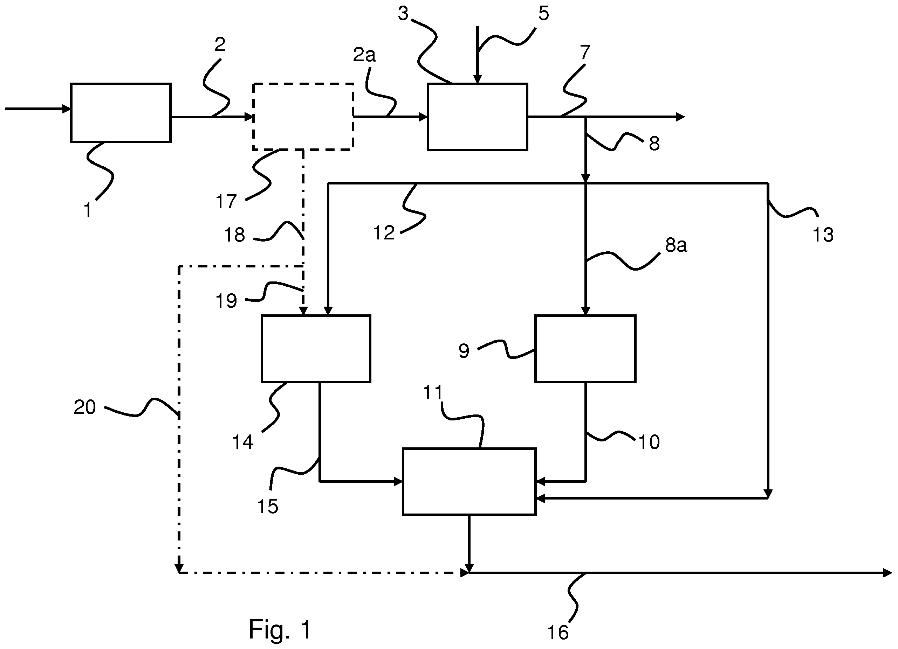

En référence à la figure 1, le procédé de production d'oligosaccharides selon l'invention comprend une étape de prétraitement de la biomasse, préalable à l'étape d'hydrolyse enzymatique, qui est réalisée dans un réacteur 1 de prétraitement. L'objectif du prétraitement est de rendre accessible la cellulose et éventuellement les hémicelluloses aux enzymes. Le prétraitement vise en particulier à modifier les propriétés physiques et physico-chimiques du matériau lignocellulosique, en vue d'améliorer l'accessibilité de la cellulose emprisonnée au sein de la matrice de lignine et d'hémicellulose.

De nombreuses technologies pour réaliser ce prétraitement existent. On peut citer par exemple la cuisson acide, la cuisson alcaline, l'explosion à la vapeur, les procédés Organosolv, etc. L'efficacité du prétraitement se mesure à la fois par le bilan matière à l'issue du prétraitement (taux de récupération des sucres sous forme de monomères ou d'oligomères solubles ou polymères insolubles) et également par la susceptibilité à l'hydrolyse enzymatique des résidus cellulosiques et hémicellulosiques.

De préférence on utilise un prétraitement par explosion à la vapeur, également connu sous le nom de "steam explosion", "steam gunning", "détente explosive", "prétraitement à la vapeur", qui se distingue par ses performances en termes de dégradabilité de la cellulose et son faible taux de dilution. Dans ce procédé la biomasse est portée rapidement à haute température (150°-250°C) par injection de vapeur sous pression. L'arrêt du traitement s'effectue généralement par décompression brutale, appelée détente ou explosion, qui déstructure la matrice lignocellulosique. Les temps de séjour varient de 10 secondes à quelques minutes, pour des pressions allant de 10 à 50 bars. Cette technique peut être mise en oeuvre soit en discontinu soit en continu. Certaines technologies proposent une injection d'eau pour refroidir le milieu avant la décompression.

L'explosion vapeur peut-être précédée d'une imprégnation d'acide pour favoriser l'hydrolyse des hémicelluloses lors de la cuisson. Lorsque l'explosion à la vapeur est appliquée sur un substrat préalablement acidifié, par exemple avec l'H₂SO₄, elle conduit à une solubilisation et une hydrolyse quasi-totale des hémicelluloses en leurs monomères, en limitant la dégradation en furfural. En outre, la susceptibilité de la cellulose à l'hydrolyse enzymatique est améliorée. L'utilisation d'un catalyseur acide permet de diminuer la température du procédé (150 à 200°C contre 250°C pour l'explosion à la vapeur sans catalyseur), et ainsi de minimiser la formation de composés de dégradation. L'explosion à la vapeur peut également être précédée d'une étape de cuisson acide qui vise à hydrolyser les hémicelluloses et à les retirer dans une solution liquide sous forme de sucres monomères et/ou d'oligomères.

L'effluent comprenant la biomasse prétraitée est soutiré par une ligne 2 et est traité dans le réacteur d'hydrolyse enzymatique 3 afin d'obtenir des sucres fermentescibles par action des cellulases sur la cellulose rendue accessible par l'étape de prétraitement. Comme montré sur la figure 1, les cellulases sont introduite dans l'unité via la ligne 5. De préférence les cellulases utiles pour la conversion de la cellulose en sucres monomères (e.g. le glucose) font parti d'un système multi-enzymatique qui comprend généralement:
- des endoglucanases (EG) qui coupent la cellulose aléatoirement au niveau des zones amorphes de la cellulose;
- des exoglucanases qui agissent de façon progressive sur les extrémités libres des chaines de cellulose;
- des β-glucosidases qui hydrolysent notamment les cellodextrines et le cellobiose en glucose.

Les conditions de l'hydrolyse enzymatique, principalement le taux de matière sèche du mélange à hydrolyser (déterminé selon la méthode ASTM E1756-01) et la quantité d'enzymes utilisée, sont choisies de telle façon que l'on obtienne une solubilisation de la cellulose comprise entre 20% et 99% et de manière préférée entre 30% et 95% poids par rapport au poids total de cellulose, de manière à fournir un hydrolysat contenant des sucres monomères comprenant notamment du glucose. L'eau nécessaire à l'obtention du taux de matière sèche visé est ajoutée par une conduite (non représentée) dans le réacteur 3. Le taux de matière sèche souhaité est généralement compris entre 5% poids et 45% poids et préférentiellement entre 8% poids et 40% poids. L'hydrolyse enzymatique est préférentiellement réalisée à pH compris entre 4 et 5,5 et à une température comprise entre 40°C et 60°C. Les additifs nécessaires, par exempleles nutriments, des réactifs chimiques comme par exemple la soude et/ou l'ammoniaque et/ou la potasse, sont introduits par une conduite (non représentée) dédiée à cet effet.

L'hydrolysat issu du réacteur d'hydrolyse enzymatique comprend une fraction liquide contenant de l'eau, un mélange de sucres monomères dont du glucose, un cocktail enzymatique et une fraction solide comprenant des matières solides non hydrolysées (dont de la lignine). De préférence l'hydrolysat qui est mise en oeuvre dans la suite du procédé présente une activité β-glucosidase d'au moins 1 IU/mL, et de préférence d'au moins 5 IU/mL. Généralement la teneur en eau de la fraction liquide de l'hydrolysat issu de l'étape d'hydrolyse est comprise entre 85 et 95% par rapport au poids total de la fraction liquide de l'hydrolysat.

Comme indiqué à la figure 1, l'hydrolysat est évacué dudit réacteur 3 par le conduit 7 et une portion (ou fraction) de l'hydrolysat est envoyée, via la ligne 8, dans une unité de traitement permettant de réduire la teneur en eau de la fraction liquide de l'hydrolysat de sorte à amener cette teneur à une valeur inférieure ou égale à 65% poids par rapport au poids total de la fraction liquide, de préférence inférieure à 60% poids, et de manière encore plus préférée inférieure à 50%.

La teneur en eau de la fraction liquide de l'hydrolysat est déterminée en filtrant un échantillon d'hydrolysat sur un milieu filtrant de porosité 1,2 µm afin de récupérer une fraction liquide. Ladite fraction liquide est ensuite soumise au test selon la norme ASTM E1756-01 qui consiste à mesurer la perte de masse de la fraction liquide par séchage à 105°C jusqu'à obtenir une masse constante du résidu La masse perdue pendant le séchage correspond ainsi à la masse d'eau présente initialement dans l'échantillon et la masse restante correspond aux matières solides et solubles contenues dans la fraction liquide.

L'étape de réduction de la teneur en eau peut être effectué par toutes les techniques de concentration connues de l'homme de l'art, comme par exemple par séparation membranaire (e.g. osmose inverse, nanofiltration), par évaporation d'une partie de l'eau, par extraction liquide-liquide. Selon une variante non représentée, la réduction de la teneur en eau est obtenue par mélange de l'hydrolysat avec une solution de sucres plus concentrée, comme par exemple un sirop de glucose.

De manière préférée, le traitement de concentration implique au moins une étape d'évaporation de l'eau. Trois modes de réalisation vont être détaillés en se référant à la figure 1.

Selon un premier mode de réalisation préféré, cette étape de réduction de la teneur en eau de l'hydrolysat est réalisée dans un évaporateur afin d'extraire l'eau et ainsi concentrer l'hydrolysat en sucres. Au moins une partie de l'hydrolysat prélevé du réacteur d'hydrolyse 3 est envoyé par la ligne 8, 8a dans un évaporateur 9 afin d'évaporer l'eau. Cette évaporation est menée en chauffant l'hydrolysat à une température inférieure à 90°C et de préférence comprise entre 40 et 70 °C sous pression atmosphérique ou sous vide. La température de mise en oeuvre est choisie de manière à ne pas dénaturer les cellulases présentes dans l'hydrolysat et qui sont utiles pour l'opération suivante d'incubation comme expliqué ci-après. L'effluent appauvri en eau issu de l'évaporateur 9 est ensuite transféré par la ligne 10 dans un réacteur d'incubation 11.

Selon un second mode de réalisation, comme indiqué sur la figure 1, l'hydrolysat qui est prélevé par la ligne 8 est divisé en deux flux respectivement par les lignes 12 et 13. Le flux d'hydrolysat soutiré par la ligne 12 est envoyé vers un évaporateur 14 qui opère à une température supérieure à 90°C, afin d'extraire l'eau et ainsi concentrer l'hydrolysat en sucres et en particulier en glucose. A l'issue de l'étape d'évaporation, l'hydrolysat est envoyé dans l'incubateur 11 grâce à la ligne 15. Quant au flux d'hydrolysat soutiré par la ligne 13, il est directement envoyé dans l'incubateur 11 de manière à apporter les cellulases nécessaires à la conversion du glucose en oligosaccharides.

Selon un troisième mode de réalisation, qui combine les deux premiers modes de réalisation décrits ci-dessus, l'hydrolysat qui est extrait par la ligne 8 est divisé en trois flux qui sont envoyés respectivement par les lignes 8a, 12 et 13 dans l'évaporateur 9, l'évaporateur 14 et l'incubateur 11.

Conformément à l'invention, le procédé de production d'oligosaccharides comprend une étape d'incubation de l'hydrolysat dont la fraction liquide comprend une teneur en eau inférieure ou égale à 65% poids. L'objectif de cette étape est de réaliser la conversion d'au moins une partie du glucose en oligosaccharides ayant des propriétés inductrices d'expression de gènes codant pour des cellulases chez des champignons. En particulier, l'étape d'incubation permet de produire du sophorose, dimère de molécules de glucoses unies par une liaison β-1-2 et/ou du gentobiose, dimère de molécules de glucoses unies par un liaison β-1-6. De préférence, la solution d'oligosaccharides contient un mélange de sophorose et de gentiobiose à une concentration totale (sophorose + gentiobiose) généralement inférieure à 30 g/L. Ces deux oligosaccharides (ou disaccharides) sont de puissants inducteurs pour la sécrétion de cellulases chez des champignons filamenteux et notamment chez *Trichoderma reesei.*

Cette étape d'incubation consiste à chauffer l'hydrolysat à une température comprise entre 40 et 70°C, de préférence entre 50 et 65°C pendant une durée comprise entre 0,1 et 100 heures, de préférence pendant au moins 24 heures, et de manière très préférée entre 24 et 72 heures.

A l'issue de l'étape d'incubation, une solution aqueuse comprenant des oligosaccharides est soutirée de l'incubateur 11 par la conduite 16.

Selon un mode de réalisation alternatif du procédé de synthèse d'oligosaccharides (non représenté à la figure 1), l'hydrolysat qui est soutiré par la conduite 8 est traité dans une unité de séparation solide/liquide d'où l'on évacue une fraction solide et une fraction liquide d'hydrolysat contenant du glucose et des cellulases. La fraction liquide d'hydrolysat est par la suite traitée selon l'un des trois modes de réalisation décrits plus haut, de sorte à en réduire sa teneur en eau avant l'étape d'incubation. Cette séparation solide/liquide peut mettre en oeuvre l'une des techniques suivantes: la centrifugation, l'essorage, le pressage, la filtration, la décantation.

En référence à la figure 1, le procédé selon l'invention peut comprendre une étape optionnelle de séparation solide/liquide, au moyen de l'unité 17, effectuée sur l'effluent issu du réacteur de prétraitement. Cette étape permet ainsi d'extraire :
- une fraction liquide contenant des sucres issus de l'hémicellulose (e.g. du xylose, arabinose, galactose et mannose) par la ligne 18; et
- une fraction contenant des solides (la biomasse prétraitée) qui est envoyée dans le réacteur d'hydrolyse 3 par la ligne 2a.

Conformément à la figure 1, tout ou partie de la fraction liquide 18 est soit directement mélangée à l'effluent issu de l'incubateur 11 via la ligne 20, soit est envoyée par la ligne 19 dans l'évaporateur 14 seule ou en mélange avec l'hydrolysat issu de l'étape d'hydrolyse afin d'y évaporer l'eau, soit (par une ligne non représentée) dans l'évaporateur 9 seule ou en mélange avec l'hydrolysat.

Selon l'invention, pour réduire la teneur en eau dans l'hydrolysat on peut mettre en oeuvre une autre méthode, alternative ou complémentaire à celles décrites plus haut, qui consiste à ajouter du sucre dans l'hydrolysat.

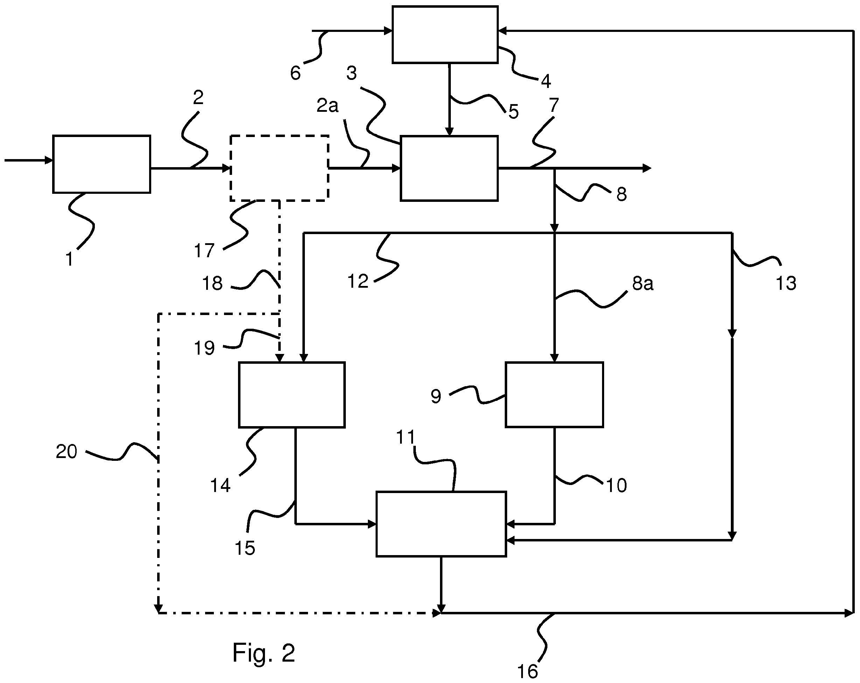

En référence à la figure 2, le procédé de production de cellulases selon l'invention met en oeuvre en plus des étapes déjà décrites plus haut, une étape de production de cellulases au moyen d'un fermenteur 4 et qui utilise les oligosaccharides qui sont synthétisés in-situ. La production d'enzymes est mise en oeuvre dans le fermenteur 4 au moyen d'un microorganisme qui est apporté par la ligne 6. Les microorganismes sont choisis de préférence parmi les bactéries cellulolytiques appartenant au genre *Ruminococus, Clostridium, Cellulomonas, Thermonospora, Streptomyces* ou parmi les champignons cellulolytiques du genre *Aspergillus, Penicillium, Trichoderma.* Parmi ceux-ci, *Trichoderma reesei* est l'espèce préférée car elle est capable de sécréter des concentrations importantes de cellulases très actives.

Comme représenté à la figure 2, tout ou partie de l'effluent qui est extrait de l'incubateur 11 par la ligne 16 est envoyé dans le fermenteur 4.

Ces souches sont cultivées en fermenteurs agités et aérés dans des conditions compatibles avec leur croissance et la production des enzymes. On peut utiliser tout type de procédé de production adapté au microorganisme connu de l'homme du métier. En particulier, on met en oeuvre un procédé de type "alimentation sans soutirage" (ou "fed-batch" selon la terminologie anglo-saxonne) comme décrit dans le document FR 2 555 603.

A cette fin, on prépare une préculture contenant le microorganisme et un milieu de culture renfermant des sels minéraux et des compléments vitaminiques usuels et une source de carbone et d'énergie de préférence sous forme de sucres solubles (par exemple le lactose, le glucose, le xylose, l'arabinose). La préculture est ensuite transférée dans un fermenteur de production d'enzymes qui comprend un milieu de culture contenant au moins un sucre et supplémenté de manière régulière avec la solution inductrice d'oligosaccharides. On procède ensuite à la production des enzymes dans le fermenteur en maintenant le contact nécessaire entre le microorganisme, le milieu de culture et en apportant régulièrement (par exemple en continu) la solution inductrice d'oligosaccharides.

La solution aqueuse contenant les oligosaccharides inducteurs est injectée, après l'épuisement de la source de carbone initiale, de façon régulière ou en continu de sorte à apporter une quantité optimisée, comprise entre 35 et 135 mg de sucres totaux (contenant les oligosaccharides inducteurs) par gramme de cellules et par heure, et de préférence entre 35 et 45 mg.

Comme indiqué sur la figure 2, tout ou partie du moût de fermentation contenant les cellulases d'intérêt est soutiré du fermenteur 4 par la ligne 5 pour alimenter le réacteur d'hydrolyse 3.

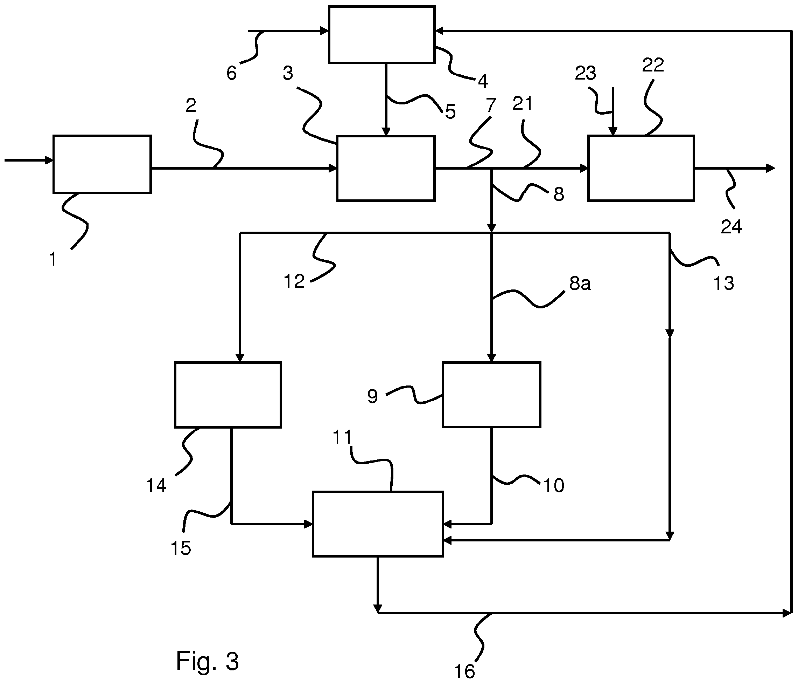

La figure 3 est une représentation d'un mode de réalisation du procédé de production d'alcools, de solvants ou d'acides organiques intégrant une unité de production de cellulases au moyen d'oligosaccharides produits à partir d'un flux interne au procédé.

En référence à la figure 3, la fraction (ou portion) de l'hydrolysat qui n'est pas utilisée pour la synthèse in-situ d'oligosaccharides est envoyée via la ligne 21 dans une unité de fermentation 22 des sucres présents dans l'hydrolysat.

Dans l'unité de fermentation 22, l'hydrolysat est mis en contact avec un ou plusieurs micro-organismes de fermentation, introduits par la ligne 23. Les sucres fermentescibles sont ainsi transformés en alcools, solvants ou acides organiques seuls ou en mélange par les micro-organismes. L'étape de fermentation dans l'unité 22 peut être réalisée à une température comprise entre 30°C et 40°C, à un pH compris entre 3 et 6,5. A l'issue de l'étape de fermentation, on obtient un moût de fermentation, évacué par le conduit 24 de l'unité 22, comprenant des matières en suspension et une phase liquide dans laquelle se trouve le ou les produits recherchés (alcools et/ou solvants).

Le moût issu de l'unité de fermentation 22 est introduit par le conduit 24 dans une unité de séparation (non représenté) qui permet de séparer le moût en différents produits : les alcools et/ou les solvants, une vinasse liquide contenant des sucres non fermentés et un résidu solide comportant principalement de la lignine, ainsi que de la cellulose, de l'hémicellulose qui n'ont pas été hydrolysées.

Par exemple la fermentation peut être du type:
a) "éthanolique" qui correspond à la production d'éthanol comme alcool majoritaire au moyen de levures (par ex., *S*. *cerevisiae*) ou de bactéries (par ex., *Z. mobilis*) ou autres micro-organismes.
b) "butylique", qui elle-même regroupe ici :
   - une fermentation produisant du n-butanol seul;
   - une fermentation "ABE" qui correspond à la production d'un mélange comprenant de l'acétone, du n-butanol (produit majoritaire), de l'éthanol. Des traces d'isopropanol peuvent également être présentes.
   - une fermentation "IBE" qui correspond à la production d'isopropanol, de n-butanol (produit majoritaire) et d'éthanol.
   Ces fermentations sont en général réalisées au moyen de microorganismes du genre *Clostridium* et sont conduites en stricte anaérobiose
   - une fermentation "isobutylique" qui correspond, en général, à la production d'isobutanol seul. De nombreux microorganismes, tous génétiquement modifiés, sont en mesure de réaliser cette conversion (par exemple *E. coli, Corynebacterium, S. cerevisiae*) à partir de la voie des acides aminés.
c) "propylique", qui correspond à la production de propanol ou d'isopropanol.

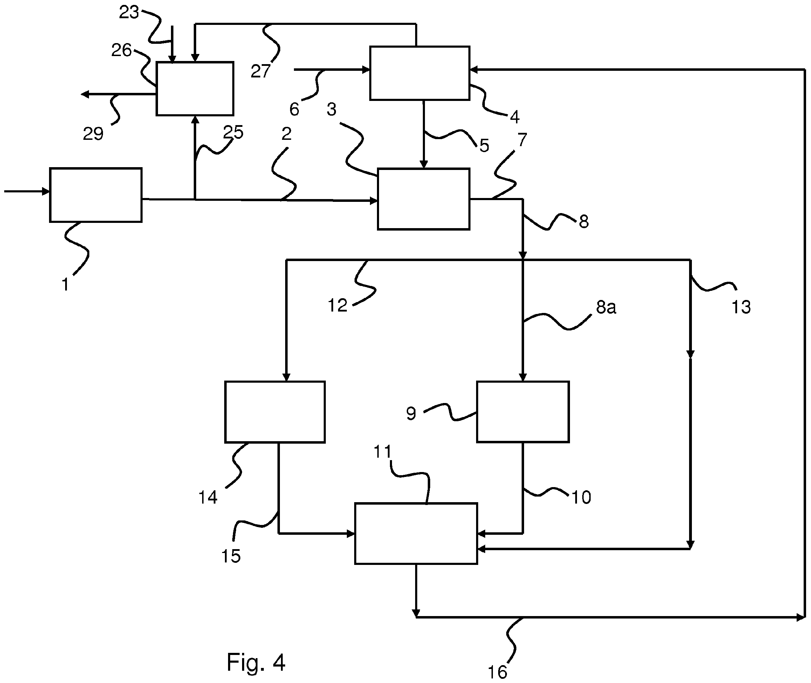

Selon un mode de réalisation du procédé de production d'alcool et/ou de solvant intégrant une unité de production de cellulases au moyen d'oligosaccharides produits à partir d'un flux interne au procédé, la fermentation des sucres en alcools et/ou solvants est réalisée de façon concomitante à l'étape d'hydrolyse enzymatique (mode de réalisation "SSF" pour *Simultaneous Saccharification and Fermentation*). En référence à la figure 4, l'effluent issu de l'unité prétraitement 1 est divisé en deux flux via les lignes 2 et 25. Le flux 2 contenant la biomasse prétraitée est envoyé dans l'unité de production in-situ de cellulase tel que déjà décrite à la figure 2. Quant au flux 25, il est transféré vers l'unité de fermentation SSF 26 qui réalise simultanément :
- l'hydrolyse de la cellulose grâce aux cellulases apportées par la ligne 27 provenant de l'unité de production in-situ de cellulases 4 et
- la fermentation des sucres fermentescibles libérés pendant l'hydrolyse enzymatique grâce aux microorganismes qui sont introduits par la ligne 23.

Lorsque l'hydrolyse enzymatique et la fermentation alcoolique sont réalisées dans une même et seule opération (procédé SSF), la température est généralement comprise entre 30 et 45°C, et le pH compris entre 4 et 6.

Il est à souligner qu'il est tout à fait possible de combiner les modes de réalisation décrits en relation avec le procédé de production d'oligosaccharides et de cellulases dans le cadre du procédé de production d'alcools et/ou de solvants selon la présente invention.

### Exemples

Dans ce qui suit, on désigne sous l'abréviation "MS" les matières sèches (solides et solubles) présentes dans un milieu. Le taux de matières sèches (ou "Total Solids") est déterminé selon la méthode ASTM E1756-01 qui consiste en une perte de masse à 105°C jusqu'à l'obtention d'une masse constante du résidu.

### Exemple 1 (non-conforme) :

Une hydrolyse enzymatique de paille prétraitée par cuisson acide est réalisée dans un milieu comprenant 15% poids de MS (15g de masse sèche de paille pour 100 g de masse totale) au moyen d'un cocktail de cellulases produit par *Trichoderma reesei.* La quantité de cellulases mise en oeuvre pour l'hydrolyse a été fixée à 10 mg de cellulases par gramme de matière sèche (MS). Le mélange est mis en hydrolyse à une température de 50°C et sous agitation pendant 72 heures. A l'issue de l'hydrolyse, on récupère un hydrolysat brut ayant une concentration en glucose de 75 g/L, une activité β-glucosidase de 10 IU/mL. En outre la fraction liquide a une teneur en eau de 92% poids par rapport au poids de la fraction liquide.

L'hydrolysat brut est ensuite mis à incuber pendant 24 heures à 50°C sous agitation. A l'issue de la phase d'incubation, l'hydrolysat est mis à bouillir pendant 5 minutes afin de dénaturer les enzymes, puis analysé par HPLC. L'analyse indique que l'hydrolysat ne contient pas d'oligosaccharides.

L'exemple 1 non-conforme à l'invention montre que l'incubation d'un hydrolysat ayant une teneur en eau supérieure à 65% poids ne permet pas de transformer au moins une partie du glucose en oligosaccharides, et en particulier en sophorose et/ou gentiobiose.

### Exemple 2 (selon l'invention) :

L'hydrolysat brut obtenu dans l'exemple 1 est complémenté avec du sirop de glucose à 60% poids afin d'obtenir une fraction liquide ayant une concentration en glucose de 500 g/L et une teneur en eau de 57,5% poids d'eau par rapport au poids total de la fraction liquide d'hydrolysat. Le mélange est mis à incuber pendant 24 heures à 50°C sous agitation. A l'issue de la phase d'incubation, le mélange est mis à bouillir pendant 5 minutes afin de dénaturer les enzymes, puis analysé par HPLC. L'hydrolysat obtenu contient 22 g/L d'un mélange de sophorose et de gentiobiose et 477 g/L de glucose (soit 4,4% de conversion du glucose en oligosaccharides).

### Exemple 3 (selon l'invention) :

L'hydrolysat brut obtenu dans l'exemple 1 est filtré au moyen d'un filtre de porosité 100µm afin de séparer :
- une fraction liquide contenant 12% poids de matière sèche (MS);
- une fraction solide contenant 35% poids de matière sèche (MS).

La fraction liquide contient 75 g/L de glucose et a une activité β-glucosidase de 6 IU/mL et une teneur en eau d'environ 90% poids.

La fraction liquide est ensuite complémentée avec du sirop de glucose à 60% poids afin d'obtenir une fraction liquide ayant une concentration en glucose de 500 g/L et une teneur en eau de 57,5% poids. Le mélange est mis à incuber pendant 24 heures à 50°C sous agitation. A l'issue de la phase d'incubation, le mélange est mis à bouillir 5 minutes afin de dénaturer les enzymes puis analysé par HPLC. L'analyse indique que l'hydrolysat contient environ 11 g/L d'un mélange de sophorose et de gentiobiose et 488 g/L de glucose (soit 2.2% de conversion du glucose en oligosaccharides).

### Exemple 4 (selon l'invention):

On prépare une solution aqueuse contenant 300 g/L de glucose, 300 g/L de xylose et 10 IU/mL d'activité β-glucosidase et dont la teneur en eau est d'environ 50% poids. Cette solution est mise à incuber pendant 24 heures à 50°C sous agitation. Après incubation, le mélange est mis à bouillir 5 minutes afin de dénaturer les enzymes puis analysé par HPLC. l'hydrolysat contient ainsi 21 g/L de mélange sophorose et gentiobiose (soit 7% de conversion du glucose en oligosaccharides), 278 g/L de glucose et 300 g/L de xylose .

Les exemples 2 à 4 selon l'invention montrent qu'il est possible de produire des oligosaccharides par incubation d'un hydrolysat de biomasse lignocellulosique lorsque sa fraction liquide a une teneur en eau inférieure à 65% poids par rapport au poids total de la fraction liquide.

L'exemple 4 indique en outre qu'il est possible de remplacer au moins une partie du glucose par des pentoses dans la solution d'incubation pour produire des oligosaccharides inducteurs.

### Exemple 5 (selon l'invention) :

Quatre bioréacteurs Dasgip de volume utile 750mL contenant un milieu salin (5 g/L KH₂PO₄, 2.8 g/L (NH₄)₂SO₄, 0.6 g/L MgSO₄,7H₂O, 0.6 g/L CaCl₂,2H₂O, 60 mg/L FeSO₄,7H₂O, 13 mg/L MnSO₄,4H₂O, 17 g/L ZnSO₄,7H₂O 1 g/L cornsteep) et 15 g/L de glucose sont stérilisés puis ensemencés avec une préculture de *Trichoderma reesei* en fiole. Tout au long de la culture, le pH est maintenu à 4.8 par ajout de base ammoniaque NH₄OH ou d'acide sulfurique H₂SO₄. Après épuisement du glucose (après environ 30 heures), une synthèse de cellulases sécrétées par *Trichoderma reesei* est réalisée en mode semi-continu (ou "fed-batch") au moyen de solutions aqueuses contenant chacune une teneur totale en sucres de 250 g/L mais de composition différentes en sucre:
- la solution 1 ne contient que du glucose seul à 250 g/L;
- la solution 2 est la fraction liquide de l'exemple 2 diluée avec de l'eau de sorte qu'elle contient 11 g/L d'un mélange sophorose et gentobiose et 239 g/L de glucose ;
- la solution 3 est la fraction liquide de l'exemple 3 diluée avec une solution aqueuse de glucose à 100 g/L, de sorte qu'elle contient 6 g/L d'un mélange sophorose et gentobiose et 244 g/L de glucose;
- la solution 4 ne contient que du lactose seul à 250 g/L.

La concentration en protéines (cellulases) dans le milieu de culture est mesurée par la méthode de Lowry [Lowry, Rosenbrough, Farr, Randall (1951) Protein measurement with the Folin phenol reagent. Journal of Biochemical Chemistry 193 (1) pages 265-275]

Avec la solution 1, on n'observe aucune induction de la production de protéines. La concentration en protéines reste stable à environ 1 à 2 g/L après 250 heures de culture.

Lorsqu'on utilise les solutions 2, 3 et 4 (selon l'invention) pour la culture, on observe une production de protéines. Après 250 heures de culture, le milieu de culture contient respectivement 35 g/L, 36 g/L et 34 g/L de protéines. Le procédé selon l'invention permet donc de produire des cellulases par des microorganismes à partir d'une solution inductrice, contenant un mélange de sophorose et de gentiobiose, obtenue à partir d'un hydrolysat de matériau lignocellulosique.

## Revendications

1. Procédé de production d'oligosaccharides à partir de biomasse lignocellulosique comprenant au moins les étapes suivantes :
a) on prétraite dans un réacteur de prétraitement la biomasse de manière à fournir un effluent contenant un substrat prétraité;
b) on effectue dans un réacteur une hydrolyse enzymatique du substrat prétraité contenu dans l'effluent issu de l'étape a) en présence de cellulases de manière à produire un hydrolysat contenant du glucose, des cellulases et d'eau;
c) on prélève au moins une partie de l'hydrolysat issu de l'étape b) comprenant une fraction liquide ;
d) on réduit la teneur en eau de ladite partie de l'hydrolysat prélevé à l'étape c) de sorte que la fraction liquide de l'hydrolysat présente une teneur en eau inférieure à 65% poids d'eau par rapport au poids total de la fraction liquide d'hydrolysat;
e) on incube, à une température comprise entre 40 et 70°C, l'hydrolysat issu de l'étape d) pendant le temps nécessaire pour produire un effluent enrichi en oligosaccharides.

2. Procédé selon la revendication 1 dans lequel à l'étape d) on concentre par évaporation à une température inférieure à 90°C l'hydrolysat prélevé à l'étape c).

3. Procédé selon la revendication 1 dans lequel à l'étape d) on ajoute du glucose dans l'hydrolysat prélevé à l'étape c).

4. Procédé selon la revendication 1 dans lequel à l'étape d) on effectue une séparation membranaire de l'hydrolysat prélevé à l'étape c) afin de séparer au moins une partie de l'eau de la fraction liquide.

5. Procédé selon la revendication 1 dans lequel à l'étape d) on sépare l'hydrolysat prélevé à l'étape c) en au moins une première portion et une seconde portion, on concentre la première portion d'hydrolysat à une température supérieure à 90°C et on mélange la portion d'hydrolysat concentré avec la seconde portion d'hydrolysat.

6. Procédé selon l'une des revendications précédentes dans lequel on effectue une séparation solide/liquide de l'effluent issu de l'étape a) de manière à récupérer une fraction liquide contenant des sucres et une fraction solide contenant le substrat prétraité, ladite fraction solide étant envoyée à l'étape b).

7. Procédé selon la revendication 6, dans lequel à l'étape d) on traite l'hydrolysat en mélange avec la fraction liquide.

8. Procédé selon la revendication 6, dans lequel on mélange la fraction liquide avec l'effluent enrichi en oligosaccharides issu de l'étape e).

9. Procédé selon l'une des revendications précédentes dans lequel l'effleuent issu de l'étape e) comprend du sophorose et/ou du gentobiose.

10. Procédé de production de cellulases à partir de biomasse lignocellulosique comprenant un procédé de production d'oligosaccharides selon l'une des revendications 1 à 9 et en outre les étapes:
f) on envoie au moins une partie de l'hydrolysat enrichi en oligosaccharides dans un réacteur contenant un milieu de culture et des microorganismes capables de produire les cellulases; et
g) on met en culture le mélange obtenu à l'étape f) de manière à produire un effluent enrichi en cellulases.

11. Procédé de production de cellulases selon la revendication 10 dans lequel le microorganisme mis en oeuvre à l'étape f) est du genre *Trichoderma reseei.*

12. Procédé de production de cellulases selon l'une des revendications 10 ou 11 dans lequel l'étape g) est réalisée en mode semi-continu.

13. Procédé de production de cellulases selon l'une des revendications 10 à 12 dans lequel on effectue une séparation solide/liquide de l'effluent issu de l'étape g) de manière à récupérer une fraction liquide contenant les cellulases et on recycle ladite fraction liquide dans le réacteur de l'étape b).

14. Procédé de production d'alcools, de solvants ou d'acides organiques seuls ou en mélange comprenant les étapes du procédé de production de cellulases selon l'une des revendications 10 à 13 et dans lequel on envoie une partie de l'hydrolysat issu de l'étape b) dans une réacteur de fermentation comprenant des microorganismes afin de produire un moût de fermentation comprenant des alcools et/ou des solvants..

15. Procédé de production d'alcools, de solvants ou d'acides organiques seuls ou en mélange comprenant les étapes du procédé de production de cellulases selon l'une des revendications 10 à 13 et dans lequel on envoie une partie de l'effluent issu de l'étape a) dans une unité d'hydrolyse et de fermentation simultanées.

16. Procédé selon la revendication 14 ou 15 dans lequel on effectue une fermentation éthanolique afin de produire de l'éthanol comme alcool majoritaire.

17. Procédé selon la revendication 14 ou 15 dans lequel on effectue une fermentation butylique afin de produire du n-butanol seul ou en mélange avec de l'acétone ou de l'iso-propanol.

## Patentansprüche

1. Verfahren zur Herstellung von Oligosacchariden aus lignocellulosehaltiger Biomasse, umfassend mindestens die folgenden Schritte:
a) Vorbehandeln der Biomasse in einem Vorbehandlungsreaktor, um einen Abfluss zu erhalten, der ein vorbehandeltes Substrat enthält;
b) Durchführen einer enzymatischen Hydrolyse des vorbehandelten Substrats, das in dem aus Schritt a) erhaltenen Abfluss enthalten ist, in Anwesenheit von Cellulasen in einem Reaktor, um ein Hydrolysat zu erzeugen, das Glucose, Cellulasen und Wasser enthält;
c) Entnehmen mindestens eines Teils des aus Schritt b) erhaltenen Hydrolysats, das eine Flüssigfraktion enthält;
d) Reduzieren des Wassergehalts des Teils des in Schritt c) entnommenen Hydrolysats derart, dass die Flüssigfraktion des Hydrolysats einen Wassergehalt von weniger als 65 Gew.-%, bezogen auf das Gesamtgewicht der Flüssigfraktion des Hydrolysats, aufweist;
e) Inkubieren, bei einer Temperatur zwischen 40 und 70 °C, des aus Schritt d) erhaltenen Hydrolysats während einer Zeit, die notwendig ist, um einen mit Oligosacchariden angereicherten Abfluss zu erzeugen.

2. Verfahren nach Anspruch 1, wobei in Schritt d) das in Schritt c) entnommene Hydrolysat bei einer Temperatur von weniger als 90 °C durch Eindampfen konzentriert wird.

3. Verfahren nach Anspruch 1, wobei in Schritt d) Glucose dem in Schritt c) entnommenen Hydrolysat zugesetzt wird.

4. Verfahren nach Anspruch 1, wobei in Schritt d) eine Membrantrennung des in Schritt c) entnommenen Hydrolysats durchgeführt wird, um mindestens einen Teil des Wassers der Flüssigfraktion abzutrennen.

5. Verfahren nach Anspruch 1, wobei in Schritt d) das in Schritt c) entnommene Hydrolysat in mindestens eine erste Portion und eine zweite Portion getrennt wird, die erste Portion des Hydrolysats bei einer Temperatur von mehr als 90 °C konzentriert wird und die Portion des konzentrierten Hydrolysats mit der zweiten Portion des Hydrolysats gemischt wird.

6. Verfahren nach einem der vorhergehenden Ansprüche, wobei eine Fest/Flüssig-Trennung des aus Schritt a) erhaltenen Abflusses durchgeführt wird, um eine zuckerhaltige Flüssigfraktion und eine das vorbehandelte Substrat enthaltende Festfraktion zu gewinnen, wobei die Festfraktion zu Schritt b) geschickt wird.

7. Verfahren nach Anspruch 6, wobei in Schritt d) das Hydrolysat in Mischung mit der Flüssigfraktion behandelt wird.

8. Verfahren nach Anspruch 6, wobei die Flüssigfraktion mit dem mit Oligosacchariden angereicherten Abfluss, der aus Schritt e) erhalten wird, gemischt wird.

9. Verfahren nach einem der vorhergehenden Ansprüche, wobei der aus Schritt e) erhaltene Abfluss Sophorose und/oder Gentobiose enthält.

10. Verfahren zur Herstellung von Cellulasen aus lignocellulosehaltiger Biomasse, umfassend ein Verfahren zur Herstellung von Oligosacchariden nach einem der Ansprüche 1 bis 9 und ferner die Schritte:
f) Einbringen mindestens eines Teils des mit Oligosacchariden angereicherten Hydrolysats in einen Reaktor, der ein Kulturmedium und Mikroorganismen enthält, die Cellulasen produzieren können; und
g) Kultivieren der in Schritt f) erhaltenen Mischung, um einen mit Cellulasen angereicherten Abfluss zu erzeugen.

11. Verfahren zur Herstellung von Cellulasen nach Anspruch 10, wobei der in Schritt f) verwendete Mikroorganismus Trichoderma reseei ist.

12. Verfahren zur Herstellung von Cellulasen nach einem der Ansprüche 10 oder 11, wobei der Schritt g) im semikontinuierlichen Modus durchgeführt wird.

13. Verfahren zur Herstellung von Cellulasen nach einem der Ansprüche 10 bis 12, wobei eine Fest/Flüssig-Trennung des aus Schritt g) erhaltenen Abflusses durchgeführt wird, um eine Cellulasen enthaltende Flüssigfraktion zu gewinnen, und die Flüssigfraktion in den Reaktor von Schritt b) zurückgeführt wird.

14. Verfahren zur Herstellung von Alkoholen, Lösungsmitteln oder organischen Säuren, allein oder in Mischung, umfassend die Schritte des Verfahrens zur Herstellung von Cellulasen nach einem der Ansprüche 10 bis 13, und wobei ein Teil des aus Schritt b) erhaltenen Hydrolysats in einen Fermentationsreaktor eingebracht wird, der Mikroorganismen enthält, um eine Fermentationsmaische zu erzeugen, die Alkohole und/oder Lösungsmittel enthält.

15. Verfahren zur Herstellung von Alkoholen, Lösungsmitteln oder organischen Säuren, allein oder in Mischung, umfassend die Schritte des Verfahrens zur Herstellung von Cellulasen nach einem der Ansprüche 10 bis 13, und wobei ein Teil des aus Schritt a) erhaltenen Abflusses in eine Einheit zur gleichzeitigen Hydrolyse und Fermentation eingebracht wird.

16. Verfahren nach Anspruch 14 oder 15, wobei eine ethanolische Fermentation durchgeführt wird, um Ethanol als Hauptalkohol zu erzeugen.

17. Verfahren nach Anspruch 14 oder 15, wobei eine butylische Fermentation durchgeführt wird, um n-Butanol, allein oder in Mischung mit Aceton oder Isopropanol, zu erzeugen.

## Claims

1. A process for the production of oligosaccharides from lignocellulosic biomass, comprising at least the following steps:
a) pre-treating the biomass in a pre-treatment reactor in order to provide an effluent containing a pre-treated substrate;
b) carrying out an enzymatic hydrolysis of the pre-treated substrate contained in the effluent obtained from step a) in a reactor, in the presence of cellulases in a manner such that a hydrolysate containing glucose, cellulases and water is produced;
c) removing at least a portion of the hydrolysate obtained from step b) comprising a liquid fraction;
d) reducing the water content of said portion of the hydrolysate removed in step c) in a manner such that the liquid fraction of the hydrolysate has a water content of less than 65% by weight of water with respect to the total weight of the liquid fraction of the hydrolysate;
e) incubating the hydrolysate obtained from step d) at a temperature in the range 40°C to 70°C for the time necessary to produce an effluent enriched in oligosaccharides.

2. The process according to claim 1 in which in step d), the hydrolysate removed in step c) is concentrated by evaporation at a temperature of less than 90°C.

3. The process according to claim 1 in which in step d), glucose is added to the hydrolysate removed in step c).

4. The process according to claim 1 in which in step d), a membrane separation of the hydrolysate removed in step c) is carried out in order to separate at least a portion of the water of the liquid fraction.

5. The process according to claim 1 in which in step d), the hydrolysate removed in step c) is separated into at least a first portion and a second portion, the first hydrolysate portion is concentrated at a temperature of more than 90°C and the concentrated hydrolysate portion is mixed with the second hydrolysate portion.

6. The process according to one of the preceding claims, in which a solid/liquid separation of the effluent obtained from step a) is carried out in order to recover a liquid fraction containing sugars and a solid fraction containing the pre-treated substrate, said solid fraction being sent to step b).

7. The process according to claim 6, in which the hydrolysate is treated in step d) as a mixture with the liquid fraction.

8. The process according to claim 6, in which the liquid fraction is mixed with the effluent enriched in oligosaccharides obtained from step e).

9. The process according to one of the preceding claims, in which the effluent obtained from step e) comprises sophorose and/or gentiobiose.

10. A process for the production of cellulases from lignocellulosic biomass, comprising a process for the production of oligosaccharides according to one of claims 1 to 9 and the following additional steps:
f) sending at least a portion of the hydrolysate enriched in oligosaccharides to a reactor containing a culture medium and microorganisms which are capable of producing cellulases; and
g) culturing the mixture in order to produce an effluent enriched in cellulases.

11. The process for the production of cellulases according to claim 10, in which the microorganism used in step f) is from the genus *Trichoderma reesei.*

12. The process for the production of cellulases according to claim 10 or claim 11, in which step g) is carried out in semi-continuous mode.

13. The process for the production of cellulases according to one of claims 10 to 12, in which a solid/liquid separation of the effluent obtained in step g) is carried out in a manner such that a liquid fraction containing cellulases is recovered and said liquid fraction is recycled to the reactor of step b).

14. A process for the production of alcohols, solvents or organic acids, alone or as a mixture, comprising the steps of the process for the production of cellulases according to one of claims 10 to 13 and in which a portion of the hydrolysate obtained from step b) is sent to a fermentation reactor comprising microorganisms in order to produce a fermentation must comprising alcohols and/or solvents.

15. A process for the production of alcohols, solvents or organic acids, alone or as a mixture, comprising the steps of the process for the production of cellulases according to one of claims 10 to 13 and in which a portion of the effluent obtained from step a) is sent to a unit for simultaneous hydrolysis and fermentation.

16. The process according to claim 14 or claim 15, in which ethanolic fermentation is carried out in order to produce ethanol as the major alcohol.

17. The process according to claim 14 or claim 15, in which butylic fermentation is carried out in order to produce n-butanol alone or as a mixture with acetone or iso-propanol.
